(19) **Europäisches Patentamt**
**European Patent Office**
**Office européen des brevets**

(11) **EP 4 629 263 A1**

(12) **EUROPEAN PATENT APPLICATION**
published in accordance with Art. 153(4) EPC

(43) Date of publication:
08.10.2025 Bulletin 2025/41

(51) International Patent Classification (IPC):
$G21K\ 5/04^{(2006.01)}$     $G21K\ 1/093^{(2006.01)}$
$A61N\ 5/10^{(2006.01)}$

(21) Application number: 23897132.9

(22) Date of filing: 02.08.2023

(52) Cooperative Patent Classification (CPC):
A61N 5/10; G21K 1/093; G21K 5/04

(86) International application number:
PCT/JP2023/028343

(87) International publication number:
WO 2024/116472 (06.06.2024 Gazette 2024/23)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.11.2022 JP 2022192239

(71) Applicant: HITACHI HIGH-TECH CORPORATION
Tokyo 105-6409 (JP)

(72) Inventors:
• YAMADA, Takahiro
Tokyo 100-8280 (JP)
• EBINA, Futaro
Tokyo 100-8280 (JP)
• NOMURA, Takuya
Tokyo 100-8280 (JP)
• SOEDA, Seiji
Tokyo 100-8280 (JP)

(74) Representative: MERH-IP Matias Erny Reichl
Hoffmann
Patentanwälte PartG mbB
Paul-Heyse-Straße 29
80336 München (DE)

(54) **SCANNING ELECTROMAGNET CONTROL SYSTEM, SCANNING ELECTROMAGNET CONTROL METHOD, AND PARTICLE THERAPY SYSTEM**

(57) Provided is a scanning electromagnet control system capable of scanning a charged particle beam at a higher speed. A scanning electromagnet (1) deflects a charged particle beam by causing magnetic fields generated by a plurality of systems of windings (U), (V), and (W) whose terminating ends are connected to each other to act on the charged particle beam. A scanning electromagnet control system (2) applies a constant voltage to each of starting ends of the windings (U), (V), and (W) of the scanning electromagnet (1) when a direction of the charged particle beam is fixed, the constant voltage corresponding to the direction of the charged particle beam, and applies a varying voltage to each of the starting ends of the windings (U), (V), and (W) when the direction of the charged particle beam is changed, the varying voltage causing a potential difference between the starting ends of the windings (U), (V), and (W) to be larger than potential differences before and after the change of the direction.

FIG. 1

Processed by Luminess, 75001 PARIS (FR)

**Description**

Technical Field

The present disclosure relates to a scanning electromagnet control system, a scanning electromagnet control method, and a particle beam therapy system.

Background Art

Particle beam therapy, in which an affected area is irradiated with a charged particle beam such as a proton beam or a carbon ion beam, has been widely used. In a particle beam therapy system that performs particle beam therapy, a charged particle beam accelerated by an accelerator to have necessary energy is transported to an irradiation nozzle by a transport device, and an affected area is irradiated with the charged particle beam from the irradiation nozzle.

In the particle beam therapy, scanning irradiation may be performed to irradiate the affected area while changing the irradiation position of the charged particle beam. In the scanning irradiation, the depth of the irradiation position of the charged particle beam is changed by changing the energy of the charged particle beam. In addition, by generating a magnetic field in a direction crossing the charged particle beam to deflect the charged particle beam, the irradiation position is changed in a plane substantially perpendicular to the depth direction. Therefore, the accelerator is provided with a device that controls the energy of the charged particle beam, and the irradiation nozzle is provided with a scanning electromagnet that generates a magnetic field in a direction crossing the charged particle beam.

PTL 1 discloses a scanning electromagnet capable of two-dimensionally scanning a charged particle beam. The scanning electromagnet generates a magnetic field corresponding to an irradiation position by applying a desired excitation current to each of three terminals connected to three systems of windings. In the scanning electromagnet, the excitation current is changed when the irradiation position is changed.

Citation List

Patent Literature

PTL 1: JP 2021-019747 A

Summary of Invention

Technical Problem

PTL 1 discloses an excitation current corresponding to an irradiation position, but does not disclose a method of setting a voltage applied to each of the terminals when the irradiation position is changed. Since the excitation current is changed when the irradiation position is changed, a counter electromotive force caused by an inductance of a coil is generated. Therefore, if an appropriate voltage is not applied, the counter electromotive force may decrease the scanning speed or form a scanning path deviating from a desired orbit, making the time taken to change the irradiation position long. When the time taken to change the irradiation position is long, the time taken to irradiate the charged particle beam, and as a result, the burden on the patient and the throughput of the treatment are affected.

An object of the present invention is to provide a scanning electromagnet control system, a scanning electromagnet control method, and a particle beam therapy system capable of scanning a charged particle beam at a higher speed.

Solution to Problem

A scanning electromagnet control system according to an aspect of the present disclosure is a scanning electromagnet control system for controlling a scanning electromagnet that causes magnetic fields generated by a plurality of systems of windings whose one ends are connected to each other to act on a charged particle beam to deflect the charged particle beam, the scanning electromagnet control system including a control unit that applies a constant voltage to each of the other ends of the windings of the scanning electromagnet when a direction of the charged particle beam is fixed, the constant voltage corresponding to the direction of the charged particle beam, and applies a varying voltage to each of the other ends of the windings when the direction of the charged particle beam is changed, the varying voltage causing a potential difference between the other ends of the windings to be larger than potential differences before and after the change of the direction.

Advantageous Effects of Invention

According to the present invention, it is possible to scan the charged particle beam at a higher speed.

Brief Description of Drawings

[FIG. 1] FIG. 1 is a diagram illustrating a scanning electromagnet and a scanning electromagnet control system according to a first embodiment of the present disclosure.
[FIG. 2] FIG. 2 is a diagram illustrating an example of an excitation current target value table.
[FIG. 3] FIG. 3 is a diagram illustrating an equivalent circuit of a scanning electromagnet.
[FIG. 4] FIG. 4 is a diagram illustrating examples of an excitation current and an applied voltage of a winding.
[FIG. 5] FIG. 5 is a diagram illustrating a configuration of a particle beam therapy system.
[FIG. 6] FIG. 6 is a diagram for explaining an irradiation nozzle and peripheral devices thereof in more detail.
[FIG. 7] FIG. 7 is a diagram schematically illustrating a cross section of a scanning electromagnet.
[FIG. 8] FIG. 8 is a diagram for explaining scanning irradiation.
[FIG. 9] FIG. 9 is a diagram illustrating an example of a dose distribution in a depth direction in which a spread out Bragg peak is formed.
[FIG. 10] FIG. 10 is a flowchart for explaining an example of a charged particle beam irradiation process.
[FIG. 11] FIG. 11 is a timing chart for explaining an example of a charged particle beam irradiation process.

Description of Embodiments

Hereinafter, embodiments of the present disclosure will be described with reference to the drawings. In the following description, components having the same functions are denoted by the same reference numerals, and the description thereof may be omitted.

(First Embodiment)

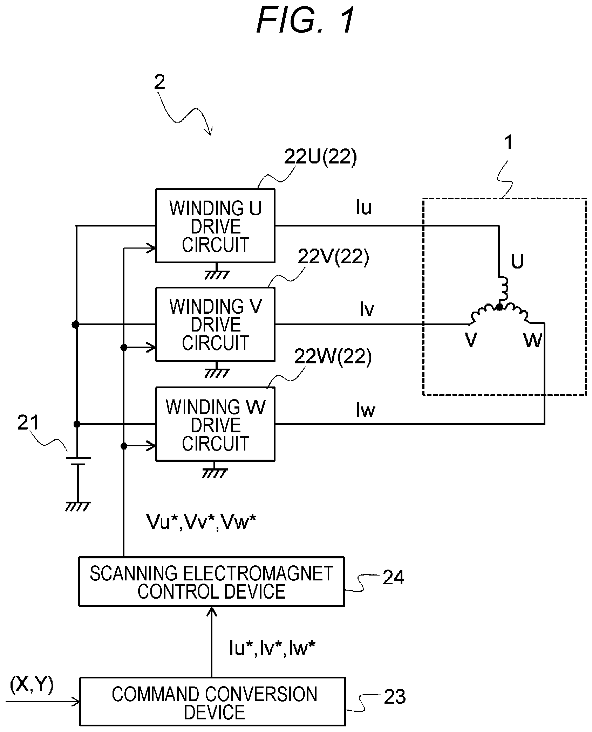

FIG. 1 is a diagram illustrating a scanning electromagnet 1 and a scanning electromagnet control system 2 according to a first embodiment of the present disclosure.
The scanning electromagnet 1 is a scanning element having a cylindrical shape and scanning a charged particle beam by deflecting the charged particle beam passing through an internal space (columnar space) thereof. Specifically, the scanning electromagnet 1 has a plurality of systems of windings, and a direction and a magnitude of a magnetic field generated in each winding are adjusted by adjusting an excitation current supplied to each winding, and accordingly, a direction of the charged particle beam is changed. In the present embodiment, the scanning electromagnet 1 has three systems of windings (windings U, V and W), and one ends (hereinafter referred to as terminating ends) of the windings U, V, and W are connected to each other.
The scanning electromagnet control system 2 is a control device that controls the scanning electromagnet 1 to scan a charged particle beam. The scanning electromagnet control system 2 includes a DC power supply 21, a winding U drive circuit 22U, a winding V drive circuit 22V, a winding W drive circuit 22W, a command conversion device 23, and a scanning electromagnet control device 24.
The DC power supply 21 is a power supply unit that generates power for applying an excitation current to each of the windings U, V, and W of the scanning electromagnet 1. In the present embodiment, an excitation current is applied to each of the windings U, V, and W of the scanning electromagnet 1 by a single DC power supply 21. A negative electrode terminal of the DC power supply 21 is connected to a ground conductor, and a positive electrode terminal of the DC power supply 21 is connected to the winding U drive circuit 22U, the winding V drive circuit 22V, and the winding W drive circuit 22W. The DC power supply 21 supplies power for applying excitation currents to the windings U, V, and W to the winding U drive circuit 22U, the winding V drive circuit 22V, and the winding W drive circuit 22W.
The winding U drive circuit 22U, the winding V drive circuit 22V, the winding W drive circuit 22W, the command conversion device 23, and the scanning electromagnet control device 24 constitute a control unit that controls the excitation currents of the windings U, V, and W using the power from the DC power supply 21.
The winding U drive circuit 22U, the winding V drive circuit 22V, and the winding W drive circuit 22W are connected to starting ends that are end portions (the other ends) different from the terminating ends of the windings U, V, and W, respectively. Each of the winding U drive circuit 22U, the winding V drive circuit 22V, and the winding W drive circuit 22W is connected to the ground conductor. Hereinafter, the winding U drive circuit 22U, the winding V drive circuit 22V, and the winding W drive circuit 22W may be collectively referred to as winding drive circuits 22.

The winding drive circuits 22 drive the windings U, V, and W of the scanning electromagnet 1 using the power supplied from the DC power supply 21 to generate scanning electromagnetic fields which are magnetic fields for causing the windings U, V, and W to deflect a charged particle beam. In the present embodiment, the winding drive circuits 22 apply excitation currents to the windings U, V, and W by applying voltages to the starting ends of the respective windings U, V, and W by pulse width modulation (PWM) control.

The command conversion device 23 and the scanning electromagnet control device 24 may be constituted by a processor. The scanning electromagnet control device 24 may further include a digital circuit. For example, the processor constituting the scanning electromagnet control device 24 controls the digital circuit by executing a program stored in a memory included in the scanning electromagnet control system 2 or a program read from the outside, thereby controlling the winding drive circuits 22 via the digital circuit.

An irradiation pattern file indicating an irradiation pattern for irradiating a charged particle beam is input from a high-order control system (not illustrated) to the command conversion device 23. The irradiation pattern file indicates, for each irradiation spot to be irradiated with the charged particle beam, an energy of the charged particle beam with which the irradiation spot is to be irradiated and an xy coordinate value (X,Y) that is a coordinate value in the x direction and the y direction of an irradiation position, which is a position of the irradiation spot, as the irradiation pattern. In the present embodiment, a depth direction is defined as a z direction, and directions substantially perpendicular to the z direction are defined as an x direction and a y direction. As will be described later, the irradiation position of the irradiation spot in the z direction is determined by the energy of the charged particle beam.

The command conversion device 23 calculates target currents, which are target values of excitation currents to be supplied to the windings U, V, and W, for each irradiation spot, based on the irradiation pattern file, and holds the calculated target currents as an excitation current target value table. In addition, the command conversion device 23 inputs the excitation current target value table to the scanning electromagnet control device 24.

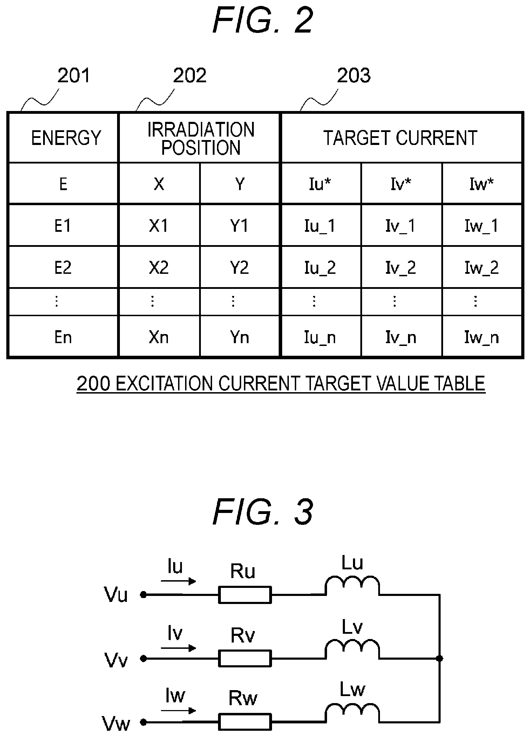

FIG. 2 is a diagram illustrating an example of an excitation current target value table. An excitation current target value table 200 illustrated in FIG. 2 has a record for each irradiation spot, and each record has fields 201 to 203. The field 201 stores an energy of a charged particle beam with which an irradiation spot is to be irradiated. The field 202 stores an xy coordinate value (X,Y) of the irradiation spot. The field 203 stores target currents that are target values of excitation currents to be supplied to the windings U, V, and W at the irradiation spot. Note that the excitation currents to be supplied to the windings U, V, and W may be denoted by Iu, Iv, and Iw, respectively, and the target values thereof may be denoted by Iu*, Iv*, and Iw*.

Referring back to FIG. 1, the description will be made. The scanning electromagnet control device 24 controls the winding drive circuits 22 based on the excitation current target value table from the command conversion device 23. Specifically, based on the excitation current target value table, the scanning electromagnet control device 24 determines target voltages that are target values of applied voltages, which are voltages to be applied to the respective starting ends of the windings U, V, and W, and causes the winding drive circuits 22 to apply the voltages having the target values to the windings U, V, and W.

Hereinafter, a determination method for determining target voltages will be described in more detail.

FIG. 3 is a diagram for explaining a method of determining applied voltages, and illustrates an equivalent circuit of the scanning electromagnet 1. As illustrated in FIG. 3, self-inductances of the windings U, V, and W are denoted by Lu, Lv, and Lw, respectively, and resistances of the windings U, V, and W are denoted by Ru, Rv, and Rw, respectively. In addition, applied voltages applied to the starting ends of the windings U, V, and W are denoted by Vu, Vv, and Vw, and applied currents (excitation currents) supplied to the windings U, V, and W are denoted by Iu, Iv, and Iw. The self-inductances and the resistances are circuit constants.

The scanning electromagnet control device 24 determines, as the target voltages, target voltages during spot stopping that are values for stopping the irradiation position by fixing the direction of the charged particle beam, and target voltages during spot moving that are values for moving the irradiation position by changing the direction of the charged particle beam. The target voltage during spot stopping is a constant voltage corresponding to the direction of the fixed charged particle beam. The target voltage during spot moving is a varying voltage that fluctuates with time, and is a voltage at which a potential difference between the starting ends of the windings U, V, and W is greater than potential differences before and after the direction of the charged particle beam is changed, that is, before and after the irradiation position is moved.

First, a method of determining target voltages during spot stopping will be described. In order to stop the irradiation position, it is necessary to make scanning electromagnetic fields excited by the scanning electromagnet 1 constant, and thus, it is also necessary to make applied current constants. Accordingly, the target voltages during spot stopping are also constant. In this case, relationships between the applied voltages and the applied currents are expressed by the following Equations 1 to 3 using the self-inductances and the resistances of the windings U, V, and W.

$$(\text{Equation } 1) \qquad Vu - Vv = Ru \cdot Iu - Rv \cdot Iv$$

$$\text{(Equation 2)} \qquad Vv - Vw = Rv \cdot Iv - Rw \cdot Iw$$

$$\text{(Equation 3)} \qquad Vw - Vu = Rw \cdot Iw - Ru \cdot Iu$$

The scanning electromagnet control device 24 determines target voltages during spot stopping, for each irradiation spot, by inputting target currents corresponding to the irradiation spot as applied currents to Equations 1 to 3. Since the self-inductances and the resistances are circuit constants, the self-inductances and the resistances are common to each irradiation spot. Since only the potential differences between the terminals of the windings U, V, and W are determined from Equations 1 to 3, the scanning electromagnet control device 24 determines target voltages during spot stopping by separately setting a reference potential. A method of setting a reference potential is not particularly limited, and for example, a potential of a terminal having the lowest potential may be set as a ground potential, a potential of a terminal having the highest potential may be set as a potential on the positive electrode side of the DC power supply 21, or an intermediate potential may be set as a ground potential.

Next, a method of determining target voltages during spot moving will be described. Since the target currents corresponding to each irradiation spot are different for each irradiation spot, it is necessary to change the applied currents by changing the voltages applied to the windings U, V, and W in order to move the irradiation position. Therefore, when the irradiation position is moved, counter electromotive forces are induced by the windings U, V, and W. The counter electromotive forces vu, vv, and vw induced by the windings U, V, and W are expressed by the following Equations 4 to 6.

$$vu = -Lu \cdot \Delta Iu/\Delta t + M \cdot (\Delta Iv/\Delta t + \Delta Iw/\Delta t) \qquad \text{(Equation 4)}$$

$$vv = -Lv \cdot \Delta Iv/\Delta t + M \cdot (\Delta Iw/\Delta t + \Delta Iu/\Delta t) \qquad \text{(Equation 5)}$$

$$vw = -Lw \cdot \Delta Iw/\Delta t + M \cdot (\Delta Iu/\Delta t + \Delta Iv/\Delta t) \qquad \text{(Equation 6)}$$

Here, $\Delta t$ represents a movement time related to the movement of the irradiation position, M represents a mutual inductance between the windings U, V, and W, and $\Delta Iu$, $\Delta Iv$, and $\Delta Iw$ represent differences in the applied currents Iu, Iv, and Iw between before and after the movement of the irradiation position. Note that the mutual inductance is common between the windings U, V, and W, but may be a different value for each combination between the windings U, V, and W.

Further, voltages of the windings U, V, and W before the movement of the irradiation position is started are denoted by Vu_0s, Vv_0s, and Vw_0s, respectively, and voltages of the windings U, V, and W at the time of starting the movement of the irradiation position (immediately after the movement is started) are denoted by Vu_0f, Vv_0f, and Vw_0f, respectively. Similarly, voltages of the windings U, V, and W after the movement of the irradiation position is completed are denoted by Vu_1s, Vv_1s, and Vw_1s, respectively, and voltages of the windings U, V, and W at the time of completing the movement of the irradiation position (immediately before the movement is completed) are denoted by Vu_1f, Vv_1f, and Vw_1f, respectively. In this case, potential differences V1 to V3 between the windings U, V, and W at the time of starting the movement of the irradiation position and potential differences V4 to V6 between the windings U, V, and W at the time of completing the movement of the irradiation position are expressed by the following Equations 7 to 12.

$$\text{(Equation 7)} \qquad V1 = Vu\_0f - Vv\_0f$$

$$\text{(Equation 8)} \qquad V2 = Vv\_0f - Vw\_0f$$

$$\text{(Equation 9)} \qquad V3 = Vw\_0f - Vu\_0f$$

$$\text{(Equation 10)} \qquad V4 = Vu\_1f - Vv\_1f$$

$$\text{(Equation 11)} \qquad V5 = Vv\_1f - Vw\_1f$$

$$\text{(Equation 12)} \qquad V6 = Vw\_1f - Vu\_1f$$

These potential differences V1 to V6 can be calculated from the following Equations 13 to 18.

(Equation 13)    V1 = Ru • Iu_0 - Rv • Iv_0-vu + vv

(Equation 14)    V2 = Rv • Iv_0 - Rw • Iw_0 - vv + vw

(Equation 15)    V3 = Rw • Iw_0 - Ru • Iu_0 - vw + vu

(Equation 16)    V4 = Ru • Iu_1 - Rv • Iv_1 - vu + vv

(Equation 17)    V5 = Rv • Iv_1 - Rw • Iw_1 - vv + vw

(Equation 18)    V6 = Rw • Iw_1 - Ru • Iu_1 - vw + vu

Here, Iu_0, Iv_0, and Iw_0 are applied currents (target currents) flowing through the windings U, V, and W before the movement of the irradiation position, and Iu_1, Iv_1, and Iw_1 are applied currents (target currents) flowing through the windings U, V, and W after the movement of the irradiation position. In addition, the first two terms on the right side in each of Equations 13 to 18 are constant terms determined from the applied currents before and after the movement of the irradiation position, and the last two terms are transient terms determined from the speeds at which the irradiation position is changed ($\Delta Iu/\Delta t$, $\Delta Iv/\Delta t$, and $\Delta Iw/\Delta t$), that is, the scanning speed at which the direction of the charged particle beam is changed.

Using Equations 13 to 18, the scanning electromagnet control device 24 determines the order of absolute values of potential differences between the starting ends of the windings U, V, and W. Then, the scanning electromagnet control device 24 determines the scanning speed by determining the change time $\Delta t$ such that a potential difference Vm having the maximum absolute value among the potential differences V1 to V6 coincides with a predetermined value. Accordingly, the scanning electromagnet control device 24 can determine absolute values of potential differences between the starting ends of the windings U, V, and W at the movement start time and at the movement completion time. Further, the scanning electromagnet control device 24 determines an absolute value of a potential of each terminal by setting a reference potential. Then, the scanning electromagnet control device 24 determines voltages of the respective starting ends of the windings U, V, and W during the movement of the irradiation position between the movement start time and the movement completion time by linearly interpolating the voltages of the starting ends at the movement start time and the voltages of the starting ends at the movement completion time. As a result, the scanning electromagnet control device 24 can realize linear scanning in which the excitation current flowing through the starting end of each of the windings U, V, and W changes at a constant rate with time from the movement start time to the movement end time. The predetermined value is, for example, an input voltage Vin from the DC power supply 21 (a voltage on the positive electrode side of the DC power supply 21).

The scanning electromagnet control device 24 may determine the change time $\Delta t$ from a preset scanning speed. In this case, the scanning electromagnet control device 24 determines the potential differences V1 to V6 between the terminals at the movement start time and at the movement completion time according to Equations 4 to 18. Further, the scanning electromagnet control device 24 determines an absolute value of a potential of each terminal by setting a reference potential. The potential during the movement can realize linear scanning at a set scanning speed by linearly interpolating the values before and after the movement.

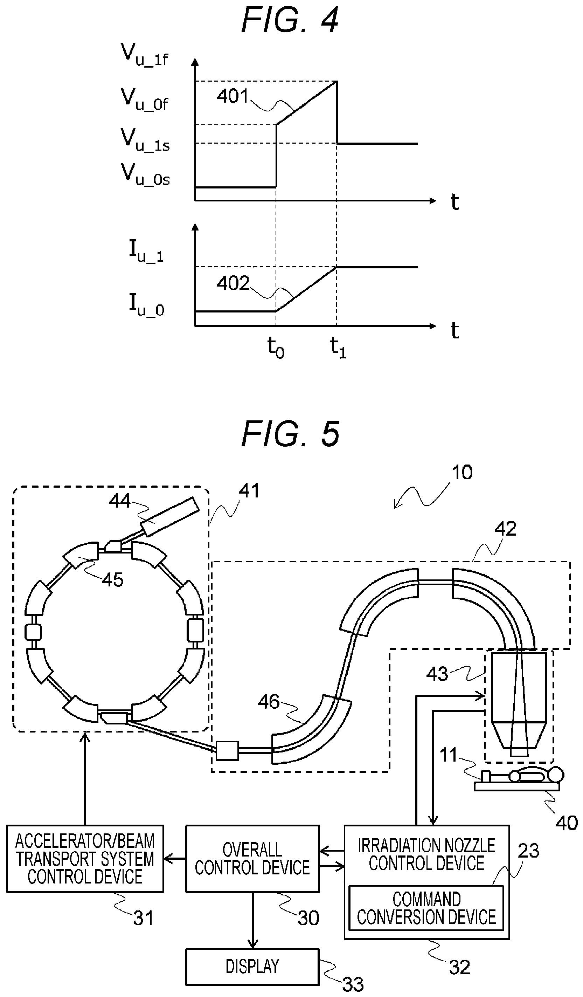

FIG. 4 is a diagram illustrating examples of an applied current and an applied voltage of the winding U determined by the above-described methods. As illustrated in FIG. 4, the applied voltage (target voltage during spot moving) 401 applied to the winding U during the movement of the irradiation position is larger than the applied voltages (target voltages during spot stopping) Vu_0s and Vu_01 before and after the movement of the irradiation position. In addition, the applied current 402 during the movement changes at a constant rate with time, that is, linearly changes. Therefore, the scanning electromagnet control device 24 determines a target voltage during movement so that the applied current during the movement of the irradiation position changes linearly.

Next, a particle beam therapy system using the scanning electromagnet 1 will be described.

FIG. 5 is a diagram illustrating a configuration of a particle beam therapy system. A particle beam therapy system 10 illustrated in FIG. 5 is a system that irradiates an affected area of a patient 11, who is a subject to be irradiated, with a charged particle beam.

The particle beam therapy system 10 includes a treatment couch 40 on which the patient 11 is placed, an accelerator 41 that accelerates a charged particle beam, a beam transport device 42 that is a transport device transporting the charged particle beam accelerated by the accelerator 41, and an irradiation nozzle 43 that irradiates an affected area of the patient 11 on the treatment couch 40 with the charged particle beam transported by the beam transport device 42.

The accelerator 41 includes an injector 44 and a synchrotron accelerator 45. The injector 44 injects charged particles into the synchrotron accelerator 45. The synchrotron accelerator 45 accelerates the charged particles injected from the injector 44 to about 60% to 70% of the velocity of light, and extracts the accelerated charged particles to the outside as a charged particle beam. The beam transport device 42 includes a deflection electromagnet 46 for deflecting the charged particle beam, and transports the charged particle beam extracted from the accelerator 41 to the irradiation nozzle 43 while the charged particle beam is deflected by the deflection electromagnet 46. The irradiation nozzle 43 irradiates an affected area of the patient 11 on the treatment couch 40 with the charged particle beam from the beam transport device 42.

The particle beam therapy system 10 includes an overall control device 30, an accelerator/beam transport system control device 31, an irradiation nozzle control device 32, and a display 33 as a control system for controlling the irradiation of the charged particle beam.

The overall control device 30, the accelerator/beam transport system control device 31, and the irradiation nozzle control device 32 may be configured by a processor. In this case, the processor constituting the overall control device 30, the accelerator/beam transport system control device 31, and the irradiation nozzle control device 32 execute various types of processes for controlling the irradiation of the charged particle beam by executing a program stored in a memory (not illustrated) included in the particle beam therapy system 10 or a program read from the outside.

The overall control device 30 controls the entire particle beam therapy system 10. The accelerator/beam transport system control device 31 controls the accelerator 41 and the beam transport device 42 according to an instruction from the overall control device 30. The irradiation nozzle control device 32 controls the irradiation nozzle 43 according to an instruction from the overall control device 30. The irradiation nozzle control device 32 includes the command conversion device 23 illustrated in FIG. 1. The display 33 displays various types of information indicating an operation state of the particle beam therapy system 10 and the like.

Note that FIG. 5 illustrates main components of the particle beam therapy system 10, and the particle beam therapy system 10 can include various devices such as peripheral devices in addition to the components illustrated in FIG. 5.

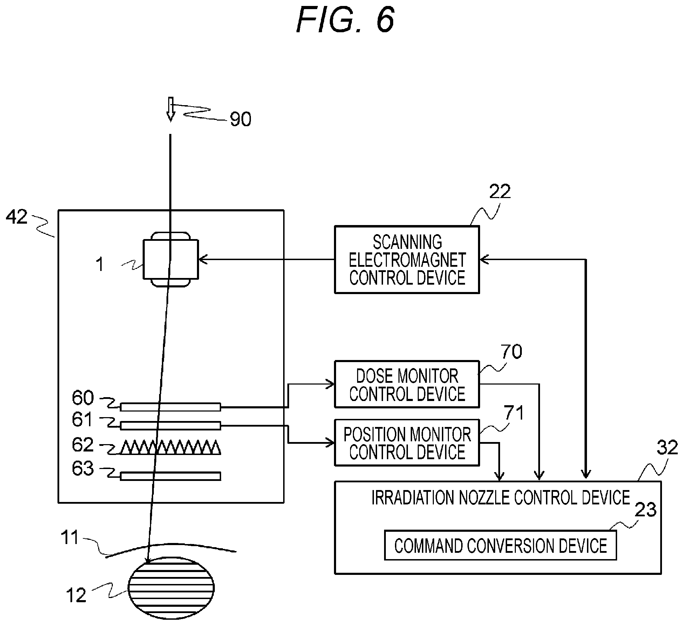

FIG. 6 is a diagram for explaining the irradiation nozzle 43 and peripheral devices thereof in more detail.

As illustrated in FIG. 6, the irradiation nozzle 43 includes a scanning electromagnet 1, a dose monitor 60, a position monitor 61, a ridge filter 62, and a range shifter 63. In addition, a charged particle beam 90 is injected into the irradiation nozzle 43.

The scanning electromagnet 1 scans the charged particle beam 90 in a two-dimensional plane substantially perpendicular to the traveling direction of the charged particle beam 90, and irradiates an affected area 12 of the patient 11 with the scanned charged particle beam 90 via the dose monitor 60, the position monitor 61, the ridge filter 62, and the range shifter 63.

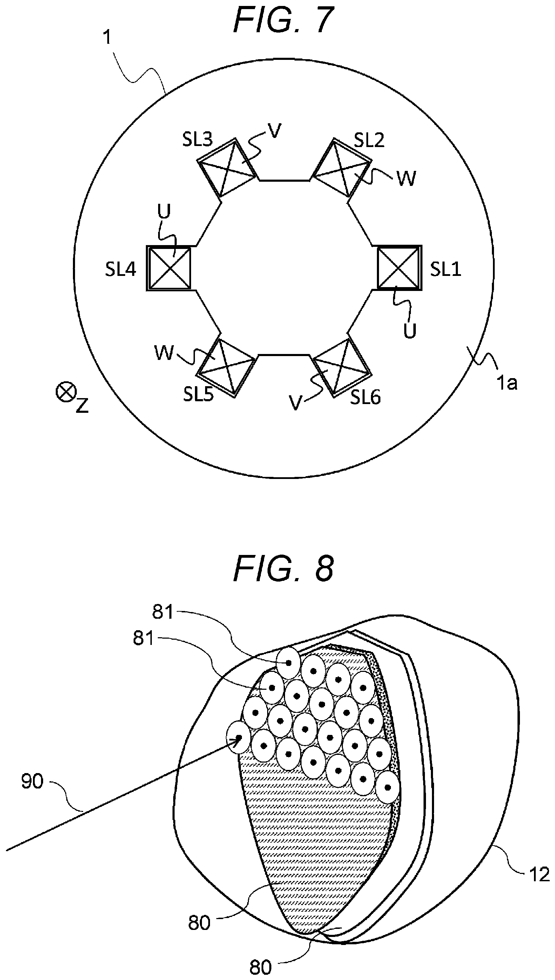

FIG. 7 is a diagram schematically illustrating a cross section of the scanning electromagnet 1 cut along a plane substantially perpendicular to the traveling direction of the charged particle beam 90, when viewed from upstream of the orbit of the charged particle beam 90. In FIG. 2, the traveling direction of the charged particle beam 90 is a Z direction.

As illustrated in FIG. 7, the scanning electromagnet 1 includes a yoke 1a having a cylindrical shape and windings U, V, and W provided inside the yoke 1a. The yoke 1a is made of a magnetic material such as iron.

In the example of FIG. 7, grooves SL1 to SL6 recessed outward and extending in the z direction are formed at 60° intervals counterclockwise in this order on an inner wall surface of the yoke 1a. The windings U, V, and W are provided in the grooves SL1 to SL6. For example, the winding U is provided in the grooves SL1 and SL4 provided at positions facing each other, the winding V is provided in the grooves SL3 and SL6 provided at positions facing each other, and the winding W is provided in the grooves SL2 and SL5 provided at positions facing each other.

Referring back to FIG. 6, the description will be made. The dose monitor 60 is a monitor for measuring a dose of the charged particle beam 90, detects electrons generated when the charged particle beam 90 passes therethrough, and outputs a pulse signal corresponding to the detected electrons as a detection signal.

The position monitor 61 is a monitor for measuring an irradiation position of the charged particle beam 90, detects electrons generated when the charged particle beam 90 passes therethrough for each passage position of the charged particle beam 90 in the position monitor 61, and outputs a pulse signal corresponding to the detected electrons as a detection signal for each passage position.

The ridge filter 62 is a filter for expanding a Bragg peak, which is an energy peak of the charged particle beam 90 in the patient 11, in the depth direction. The range shifter 63 is a plate for adjusting the maximum length that the charged particle beam 90 reaches. The range shifter 63 is inserted into the scanning electromagnet 1 as necessary.

A dose monitor control device 70 and a position monitor control device 71 are connected to the dose monitor 60 and the position monitor 61, respectively. The dose monitor control device 70 and the position monitor control device 71 may be constituted by a processor. In this case, the processor constituting the dose monitor control device 70 and the position monitor control device 71 executes various types of processes by executing a program stored in a memory included in the particle beam therapy system 10 or a program read from the outside.

Based on the detection signal output from the dose monitor 60, the dose monitor control device 70 measures an irradiation dose of the charged particle beam 90, and outputs a dose signal indicating the irradiation dose. Based on the detection signal output from the position monitor 61, the position monitor control device 71 measures a passage position at which the charged particle beam 90 has passed through the position monitor 61, and outputs a position signal indicating the passage position.

The dose monitor control device 70 and the position monitor control device 71 are connected to the irradiation nozzle control device 32. Based on the detection signal from the dose monitor control device 70, the irradiation nozzle control device 32 acquires an irradiation dose of the charged particle beam 90. Further, based on the detection signal from the position monitor control device 71, the irradiation nozzle control device 32 acquires an irradiation position of the charged particle beam 90 by calculating a position and a width of an irradiation spot 81. The irradiation nozzle control device 32 controls the irradiation of the charged particle beam 90 based on the irradiation dose and the irradiation position.

Next, the operation of the particle beam therapy system 10 will be described. In the present embodiment, the particle beam therapy system 10 irradiates the charged particle beam 90 by scanning irradiation.

FIG. 8 is a diagram for explaining scanning irradiation. As illustrated in FIG. 8, in the scanning irradiation, the affected area 12 is divided into a plurality of layers 80 arranged in the depth direction, and one or more irradiation spots 81 are arranged in one layer 80. In addition, a treatment planning device (not illustrated) calculates a position of each irradiation spot 81 and a target irradiation amount for each irradiation spot 81 to generate treatment plan information so that the affected area 12 is irradiated with a uniform dose. For each layer 80, the particle beam therapy system 10 sequentially irradiates the irradiation spots 81 in the layer 80 with the charged particle beam 90 in a predetermined order.

When the energy of the charged particle beam 90 changes, the arrival position of the charged particle beam 90 changes. Specifically, the higher the energy, the deeper the charged particle beam 90 reaches in the body. Therefore, the irradiation position in the depth direction of the affected area 12 is changed by changing the energy of the charged particle beam 90. Accordingly, the energy of the charged particle beam 90 varies for each layer 80, but is the same within the same layer 80.

In the present embodiment, the affected area 12 is irradiated with the charged particle beam to form a spread out Bragg peak (SOBP). The SOBP is realized by appropriately allocating the irradiation dose to each of a plurality of charged particle beams having different energies, and has a substantially uniform dose distribution in the depth direction.

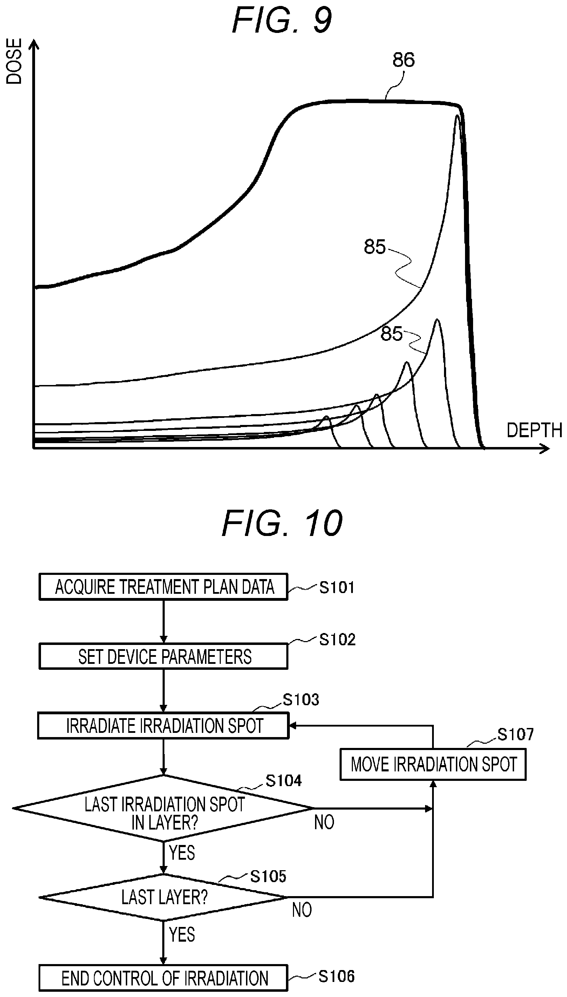

FIG. 9 is a diagram illustrating an example of a dose distribution in a depth direction in which a spread out Bragg peak is formed.

As illustrated in FIG. 9, the irradiation dose is appropriately allocated to each of the plurality of charged particle beams having different energies, so that the charged particle beam having each energy is irradiated with a dose distribution indicated by a Bragg curve 85. As a result, the plurality of Bragg curves 85 corresponding to the respective energies overlap each other to form a substantially uniform dose distribution in the depth direction as indicated by an SOBP curve 86.

FIG. 10 is a flowchart for explaining an example of a charged particle beam irradiation process performed by the particle beam therapy system 10. Note that the treatment plan information generated in advance by the treatment planning device described above is transmitted from the treatment planning device to an oncology information system (OIS) (not illustrated) and stored in the OIS. Further, the treatment plan information is transmitted from the OIS to the overall control device 30 of the particle beam therapy system 10 illustrated in FIG. 9.

In step S101, the overall control device 30 receives treatment plan information from the OIS. At this time, the overall control device 30 may display the treatment plan information and information indicating the operation of the particle beam therapy system 10 and the like on the display 33.

In step S102, the overall control device 30 sets device parameters for controlling the accelerator/beam transport system control device 31, the irradiation nozzle control device 32, and the treatment couch 40 based on the treatment plan information. The overall control device 30 controls the accelerator/beam transport system control device 31, the irradiation nozzle control device 32, and the treatment couch 40 based on the device parameters.

For example, the overall control device 30 transmits an energy of a charged particle beam corresponding to each irradiation spot, an xy coordinate value (X,Y) of an irradiation position, an irradiation dose, and the like to the irradiation nozzle control device 32 as the device parameters. The irradiation nozzle control device 32 receives the device parameters and passes an irradiation pattern file corresponding to the device parameters to the command conversion device 23. The command conversion device 23 calculates target currents for the windings U, V, and W, respectively, for each irradiation spot based on the irradiation pattern file, and outputs the calculated target currents to the scanning electromagnet control device 24 as an excitation current target value table. The scanning electromagnet control device 24 calculates target voltages for the windings U, V, and W, respectively, based on the excitation current target value table. The target voltages include target voltages during spot stopping for each irradiation spot and target voltages during spot moving between the irradiation spots.

In step S103, the accelerator/beam transport system control device 31 and the irradiation nozzle control device 32 control the accelerator 41, the beam transport device 42, and the irradiation nozzle 43 according to the control of the overall control device 30. As a result, a j-th irradiation spot in an i-th layer is irradiated with the charged particle beam. At this time, the voltages applied to the starting ends of the windings U, V, and W are constant voltages corresponding to the position of the j-th irradiation spot.

i is an integer of 1 to K, and identifies a layer set in the affected area 12. j takes an integer of 1 to M(i) for each layer of the affected area 12, and identifies an irradiation spot in the layer. In the first irradiation, i=1 and j=1. The integer M(i) identifies the last irradiation spot in the i-th layer, and the integer M(K) identifies the last irradiation spot in the last layer (i=K).

When the irradiation of the j-th irradiation spot in the i-th layer with the charged particle beam is completed, step S104 is executed. In step S104, the overall control device 30 determines whether the irradiation of the last irradiation spot of the current layer with the charged particle beam has been completed. When the irradiation of the last irradiation spot of the current layer is completed, step S105 is executed, and when the irradiation of the last irradiation spot of the current layer is not completed, step S107 is executed.

In step S105, the overall control device 30 determines whether the irradiation of the last layer with the charged particle beam has been completed. When the irradiation of the last layer is not completed, step S106 is executed, and when the irradiation of the last layer is completed, step S107 is executed.

In step S106, the overall control device 30 controls the accelerator/beam transport system control device 31 and the irradiation nozzle control device 32 to perform a movement process of moving the irradiation spot. At this time, when the irradiation of the last irradiation spot in the current layer with the charged particle beam is not completed (step S104: No), the irradiation spot after the movement is a j+1-th irradiation spot in the i-th layer, and when the irradiation of the last layer with the charged particle beam is not completed (step S105: No), the irradiation spot after the movement is a first irradiation spot in an i+1-th layer. In addition, in the movement process, the voltages applied to the starting ends of the windings U, V, and W are target voltages during spot moving calculated from the target voltages during spot stopping corresponding to the irradiation spots before and after the movement.

In step S107, the overall control device 30 stops the control for the irradiation of the charged particle beam and ends the radiation therapy.

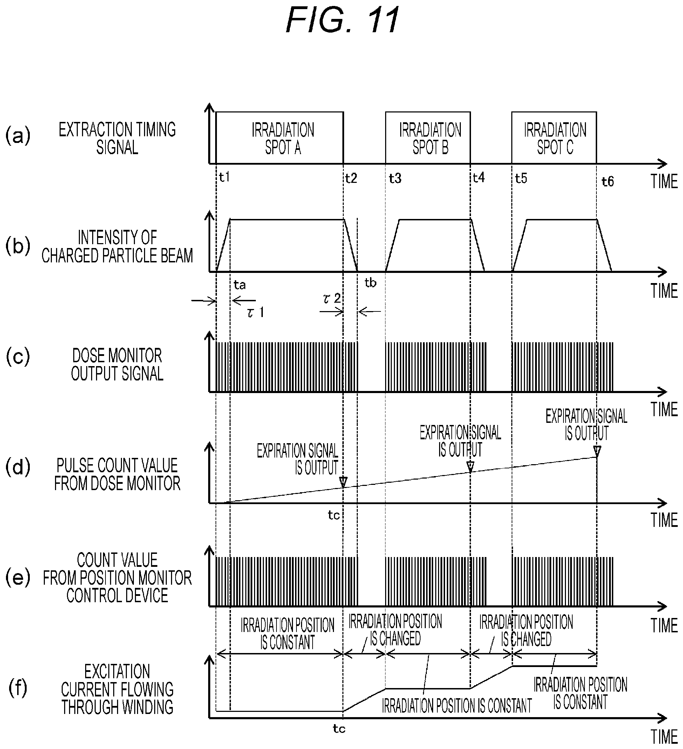

FIG. 11 is a timing chart for explaining an example of a charged particle beam irradiation process. FIG. 11 illustrates an example in which three irradiation spots from irradiation spot A to irradiation spot C are irradiated with charged particle beams.

As illustrated in FIG. 11(a), in the irradiation of the irradiation spot A, an extraction timing signal is output from the accelerator/beam transport system control device 31 to the accelerator 41 from time t1 to time t2, and the affected area 12 is irradiated with a charged particle beam. Similarly, in the irradiation of the irradiation spot B, an extraction timing signal is output from the accelerator/beam transport system control device 31 to the accelerator 41 from time t3 to time t4, and in the irradiation of the irradiation spot C, an extraction timing signal is output from the accelerator/beam transport system control device 31 to the accelerator 41 from time t5 to time t6. In this way, the irradiations of the irradiation spots A to C are performed by similar processes. Therefore, the irradiation of the irradiation spot A will be mainly described below.

At the time of irradiating the irradiation spot A, when the extraction timing signal rises at time t1 as illustrated in FIG. 11(a), the irradiation of the charged particle beam is started as illustrated in FIG. 11 (b). Thereafter, the intensity of the charged particle beam increases, and the intensity of the charged particle beam reaches the maximum value at time ta after a lapse of time $\tau$1 from time t1. Thereafter, when the extraction timing signal falls at time t2, the intensity of the charged particle beam decreases, and the intensity of the charged particle beam becomes 0 at time tb after a lapse of time $\tau$2 from time t2. That is, the irradiation of the charged particle beam ends.

When the extraction timing signal rises at time t1, as shown in FIG. 11(c), the dose monitor 60 in the irradiation nozzle 43 detects electrons generated when the charged particle beam passes therethrough, and outputs a pulse signal corresponding to the detected electrons to the dose monitor control device 70 as a detection signal. The dose monitor control device 70 counts the number of pulse signals, which are detection signals from the dose monitor 60, as a pulse count value. As a result, the pulse count value increases as illustrated in FIG. 11(d). When the pulse count value reaches a predetermined value at time tc before time t2, the dose monitor control device 70 transmits an expiration signal to that effect to the irradiation nozzle control device 32. Upon receiving the expiration signal, the irradiation nozzle control device 32 stops the irradiation of the irradiation spot A with the charged particle beam. Here, the predetermined value is determined according to a target irradiation amount for the irradiation spot A.

In addition, when the extraction timing signal rises at time t1, as illustrated in FIG. 11(e), similarly to the dose monitor 60, the position monitor 61 in the irradiation nozzle 43 detects electrons generated when the charged particle beam passes therethrough for each passage position of the charged particle beam, and outputs a pulse signal for each passage position corresponding to the detected electrons to the position monitor control device 71 as a detection signal. The position monitor control device 71 counts the number of pulse signals as a pulse count value for each passage position.

When the irradiation of the irradiation spot A is completed, the position monitor control device 71 outputs the pulse count value counted during the irradiation period for the irradiation spot A to the irradiation nozzle control device 32. The irradiation nozzle control device 32 calculates a position and a size of the irradiation spot actually irradiated with the charged particle beam based on the pulse count value from the position monitor control device 71, and determines whether the predetermined position is irradiated with the charged particle beam 90 based on the position and size. When the predetermined position is not irradiated with the charged particle beam 90, that is, when a deviation amount of the position or size of the irradiation spot exceeds a predetermined value, the overall control device 30 determines that there is a possibility that a problem has occurred and stops the irradiation of the charged particle beam.

When the irradiation nozzle control device 32 receives the expiration signal from the dose monitor control device 70, the command conversion device 23 of the irradiation nozzle control device 32 obtains target currents corresponding to an irradiation position of a next irradiation spot and transmits the obtained target currents to the scanning electromagnet control device 24. Upon receiving the target currents, the scanning electromagnet control device 24 calculates target voltages during spot stopping corresponding to the next irradiation spot and target voltages during spot moving to the next irradiation spot based on the target currents, and applies the target voltages during spot moving to the starting ends of the windings U, V, and W to control the excitation currents flowing through the windings U, V, and W.

FIG. 11(f) illustrates changes in the excitation currents flowing through the windings U, V, and W. The excitation currents are constant from time t1 when the extraction timing signal rises to time tc when the extraction timing signal starts to fall. In this time zone, an xy coordinate value (X,Y) of the irradiation position is constant. During a period from time tc to time t3 when the extraction timing signal rises for irradiating the next irradiation spot B, the excitation currents approach the target currents a constant rate with time for the next irradiation spot B, and reaches the target currents at time t3. The xy coordinate value (X,Y) of the irradiation position changes in this time zone.

Here, the irradiation of the irradiation spot A with the charged particle beam has been described, but the irradiation spot B and the irradiation spot C are also irradiated with charged particle beams by similar processes.

As described above, according to the present embodiment, the scanning electromagnet 1 causes magnetic fields generated by the plurality of systems of windings U, V, and W whose terminating ends are connected to each other to act on a charged particle beam to deflect the charged particle beam. When a direction of the charged particle beam is fixed, the scanning electromagnet control system 2 applies a constant voltage corresponding to the direction of the charged particle beam to each of starting ends of the windings U, V, and W of the scanning electromagnet 1, and when the direction of the charged particle beam is changed, the scanning electromagnet control system 2 applies a varying voltage at which a potential difference between the starting ends of the windings U, V, and W is larger than potential differences before and after the change of the direction to each of the starting ends of the windings U, V, and W. Therefore, when the direction of the charged particle beam is changed, the potential difference between the windings **U, V,** and W becomes larger than the potential differences before and after the change of the charged particle beam, making it possible to reduce the influence on the scanning speed by counter electromotive forces generated by changes in excitation currents flowing through the windings U, V, and W. Accordingly, the charged particle beam can be scanned at a higher speed.

In the present embodiment, the scanning electromagnet control system 2 determines the varying voltage for changing the direction of the charged particle beam based on excitation currents flowing through each of the windings U, V, and W according to the target voltages before and after the change of the direction of the charged particle beam and a scanning speed for changing the direction of the charged particle beam. Therefore, the influence of the counter electromotive forces on the scanning speed can be more appropriately reduced.

Further, in the present embodiment, the scanning electromagnet control system 2 determines a value of the varying voltage at the movement start time when the change of the direction of the charged particle beam is started based on a target voltage during spot stopping before the change of the direction of the charged particle beam and the scanning speed, and determines a value of the varying voltage at the movement end time when the change of the direction of the charged particle beam ends based on a target voltage during spot stopping after the change of the direction of the charged particle beam and the scanning speed. In addition, the scanning electromagnet control system 2 determines a value of the varying voltage between the movement start time and the movement end time based on the value of the varying voltage at the movement start time and the value of the varying voltage at the movement end time. Therefore, the influence of the counter electromotive forces on the scanning speed can be more appropriately reduced.

In addition, in the present embodiment, the scanning electromagnet control system 2 determines the value of the varying voltage between the movement start time and the movement end time by linearly interpolating the value of the varying voltage at the movement start time and the value of the varying voltage at the movement end time. This makes it possible to linearly change the excitation current from the movement start time to the movement end time, thereby scanning the charged particle beam along a linear orbit at a constant speed.

In the present embodiment, the scanning electromagnet control system 2 determines the value of the varying voltage between the movement start time and the movement end time such that an excitation current flowing through each of the windings U, V, and W changes at a constant rate with time between the movement start time and the movement end time. Therefore, it is possible to scan the charged particle beam along a linear orbit at a constant speed.

In the present embodiment, voltages are applied to the plurality of windings U, V, and W using the single DC power supply 21, and therefore, it is possible to reduce the influence of the counter electromotive forces on the scanning speed with a simple device configuration.

In the present embodiment, the windings are three systems of windings U, V, and W. Therefore, the charged particle beam can be two-dimensionally scanned with a simple device configuration.

(Second Embodiment)

The present embodiment is different from the first embodiment in a method of applying voltages to the windings U, V, and W during the movement of the irradiation position.

Hereinafter, a terminal on a high-potential side and a terminal on a low-potential side at two of the starting ends of the windings U, V, and W to which the potential difference Vm having the maximum absolute value among the potential differences V1 to V6 expressed by Equations 13 to 18 is applied are denoted by T1 and T2, respectively. Among the starting ends of the windings U, V, and W, the starting end other than the starting ends T1 and T2 is denoted by T3.

In the present embodiment, the scanning electromagnet control device 24 obtains the potential difference Vm having the maximum absolute value among the potential differences V1 to V6 using Expressions 13 to 18. Then, when the movement of the irradiation position is started, the scanning electromagnet control device 24 continuously connects the high-potential side of the DC power supply 21 to the starting end T1 and continuously grounds the starting end T2 for a certain time Δt'. As a result, the potential difference between the starting ends T1 and T2 is set to the potential difference of the DC power supply 21 (the potential difference between the positive electrode and the negative electrode). The scanning electromagnet control device 24 applies a voltage to the starting end T3 according to a target voltage obtained by linearly interpolating values of the varying voltages before and after the movement, similarly to the first embodiment.

After the time Δt' elapses, the scanning electromagnet control device 24 performs excitation current feedback control to adjust the value of the voltage applied to each of the starting ends of the windings U, V, and W so that the excitation current becomes the target current after the end of movement.

For example, the time Δt' may be determined based on the moving distance of the irradiation position, or may be determined as a time required until the current flowing through the predetermined starting end reaches the target current.

As described above, according to the present embodiment, since the voltage of the DC power supply 21 can be efficiently applied between the starting ends where a large potential difference is required, high-speed scanning can be performed.

Note that the present disclosure is not limited to the above-described embodiments, and includes various modifications. For example, each of the above embodiments is for describing the present disclosure in an easy-to-understand manner, and the present disclosure is not necessarily limited to having all the configurations described above.

In addition, a part of the configuration of one embodiment may be replaced with the configuration of another embodiment, and the configuration of one embodiment may be added to the configuration of another embodiment. Furthermore, a part of the configuration of each embodiment may be added to, deleted from, or replaced with another configuration.

In the above description, the discrete-irradiation spot irradiation method in which the charged particle beam is stopped between the irradiation spots is adopted, but the continuous-irradiation spot irradiation method in which the charged particle beam is not stopped between the irradiation spots may be adopted.

The accelerator 41 is not limited to the synchrotron accelerator 45, and any of the various known accelerators such as a cyclotron accelerator or a synchrocyclotron accelerator may be used. The charged particles accelerated by the accelerator 41 may be protons or heavy particles such as carbon.

The scanning electromagnet 1 according to each embodiment may be provided in the accelerator 41 and the beam transport device 42 or the like, not limited to the irradiation nozzle 43. That is, the scanning electromagnet 1 may be used for the purpose of correcting the orbit of the charged particle beam 90 in the accelerator 41 and the beam transport device 42.

The processes of the devices such as the command conversion device 23, the scanning electromagnet control device 24, the overall control device 30, the accelerator/beam transport system control device 31, and the irradiation nozzle control device 32, as well as a distribution analysis device 113, an irradiation control device 104, a data server 111, and a treatment planning device 112, may be shared by a plurality of processors or computer systems, or some physical configurations (databases and the like) may be connected thereto via a network.

The above-described embodiments of the present disclosure are examples for describing the present disclosure, and are not intended to limit the scope of the present disclosure only thereto. Those skilled in the art can embody the present disclosure in various other aspects without departing from the scope of the present disclosure.

Reference Signs List

1 scanning electromagnet
1a yoke
2 scanning electromagnet control system
10 particle beam therapy system
11 patient
12 affected area
21 DC power supply
22 winding drive circuit
22U winding U drive circuit
22V winding V drive circuit
22W winding W drive circuit
23 command conversion device
24 scanning electromagnet control device
26 DC power supply
30 overall control device
31 beam transport system control device
32 irradiation nozzle control device
33 display
40 treatment couch
41 accelerator
42 beam transport device
43 irradiation nozzle
44 injector
45 synchrotron accelerator
46 deflection electromagnet
60 dose monitor
61 position monitor
62 ridge filter
63 range shifter
70 dose monitor control device
71 position monitor control device
U to W winding

## Claims

1. A scanning electromagnet control system for controlling a scanning electromagnet that causes magnetic fields generated by a plurality of systems of windings whose one ends are connected to each other to act on a charged particle beam to deflect the charged particle beam, the scanning electromagnet control system comprising:
a control unit that applies a constant voltage to each of the other ends of the windings of the scanning electromagnet when a direction of the charged particle beam is fixed, the constant voltage corresponding to the direction of the charged particle beam, and applies a varying voltage to each of the other ends of the windings when the direction of the charged particle beam is changed, the varying voltage causing a potential difference between the other ends of the windings to be larger than potential differences before and after the change of the direction.

2. The scanning electromagnet control system according to claim 1, wherein the control unit determines the varying voltage based on currents flowing through each of the windings according to constant voltages before and after the change of the direction and a scanning speed for changing the direction.

3. The scanning electromagnet control system according to claim 2, wherein the control unit determines a value of the varying voltage at a start time when the change of the direction is started based on the constant voltage before the change of the direction and the scanning speed, determines a value of the varying voltage at an end time when the change of the direction ends based on the constant voltage after the change of the direction and the scanning speed, and determines a value of the varying voltage between the start time and the end time based on the value of the varying voltage at the start time and the value of the varying voltage at the end time.

4. The scanning electromagnet control system according to claim 3, wherein the control unit determines the value of the varying voltage between the start time and the end time by linearly interpolating the value of the varying voltage at the start time and the value of the varying voltage at the end time.

5. The scanning electromagnet control system according to claim 3, wherein the control unit determines the value of the varying voltage between the start time and the end time such that an excitation current flowing through each of the other ends changes at a constant rate with time between the start time and the end time.

6. The scanning electromagnet control system according to claim 1, further comprising:

   a DC power supply that generates a voltage to be applied to each of the windings,
   wherein the control unit sets a potential difference between the other ends of two predetermined windings of the windings to a potential difference of the DC power supply at a start time when the change of the direction is started, and then adjusts a value of the varying voltage applied to each of the windings such that a current flowing through each of the other ends of the windings becomes a target current.

7. The scanning electromagnet control system according to claim 1, further comprising:

   a single DC power supply,
   wherein the control unit applies a voltage to each of the windings using the DC power supply.

8. The electromagnet control system according to claim 1, wherein the windings are three systems of windings.

9. A scanning electromagnet control method performed by a scanning electromagnet control system for controlling a scanning electromagnet that causes magnetic fields generated by a plurality of systems of windings whose one ends are connected to each other to act on a charged particle beam to deflect the charged particle beam, the scanning electromagnet control method comprising:

   applying a constant voltage to each of the other ends of the windings of the scanning electromagnet when a direction of the charged particle beam is fixed, the constant voltage corresponding to the direction of the charged particle beam; and
   applying a varying voltage to each of the other ends of the windings when the direction of the charged particle beam is changed, the varying voltage causing a potential difference between the other ends of the windings to be larger than potential differences before and after the change of the direction.

10. A particle beam therapy system including an accelerator that extracts a charged particle beam, a transport device that transports the charged particle beam extracted from the accelerator, and an irradiation nozzle that irradiates a patient with the charged particle beam transported by the transport device, the particle beam therapy system comprising:

    a scanning electromagnet provided in at least one of the accelerator, the transport device, and the irradiation nozzle to cause magnetic fields generated by a plurality of systems of windings whose one ends are connected to each other to act on the charged particle beam to deflect the charged particle beam; and
    a scanning electromagnet control system that applies a constant voltage to each of the other ends of the windings of the scanning electromagnet when a direction of the charged particle beam is fixed, the constant voltage corresponding to the direction of the charged particle beam, and applies a varying voltage to each of the other ends of the windings when the direction of the charged particle beam is changed, the varying voltage causing a potential difference between the other ends of the windings to be larger than potential differences before and after the change of the direction.

# FIG. 1

## FIG. 2

| ENERGY | IRRADIATION POSITION | | TARGET CURRENT | | |
|:---:|:---:|:---:|:---:|:---:|:---:|
| E | X | Y | Iu* | Iv* | Iw* |
| E1 | X1 | Y1 | Iu_1 | Iv_1 | Iw_1 |
| E2 | X2 | Y2 | Iu_2 | Iv_2 | Iw_2 |
| ⋮ | ⋮ | ⋮ | ⋮ | ⋮ | ⋮ |
| En | Xn | Yn | Iu_n | Iv_n | Iw_n |

200 EXCITATION CURRENT TARGET VALUE TABLE

## FIG. 3

## FIG. 4

## FIG. 5

# FIG. 6

## FIG. 7

## FIG. 8

# FIG. 9

# FIG. 10

# FIG. 11

(a) EXTRACTION TIMING SIGNAL

IRRADIATION SPOT A    IRRADIATION SPOT B    IRRADIATION SPOT C

t1    t2    t3    t4    t5    t6    TIME

(b) INTENSITY OF CHARGED PARTICLE BEAM

ta    tb

$\tau 1$    $\tau 2$

TIME

(c) DOSE MONITOR OUTPUT SIGNAL

TIME

(d) PULSE COUNT VALUE FROM DOSE MONITOR

EXPIRATION SIGNAL IS OUTPUT    EXPIRATION SIGNAL IS OUTPUT    EXPIRATION SIGNAL IS OUTPUT

tc    TIME

(e) COUNT VALUE FROM POSITION MONITOR CONTROL DEVICE

IRRADIATION POSITION IS CONSTANT    IRRADIATION POSITION IS CHANGED    IRRADIATION POSITION IS CHANGED    TIME

(f) EXCITATION CURRENT FLOWING THROUGH WINDING

IRRADIATION POSITION IS CONSTANT    IRRADIATION POSITION IS CONSTANT

tc    TIME

## INTERNATIONAL SEARCH REPORT

International application No.

**PCT/JP2023/028343**

| A. | CLASSIFICATION OF SUBJECT MATTER |
|---|---|

*G21K 5/04*(2006.01)i; *G21K 1/093*(2006.01)i; *A61N 5/10*(2006.01)i
FI:   G21K1/093 S; A61N5/10 M; A61N5/10 H; G21K5/04 A; G21K5/04 C

According to International Patent Classification (IPC) or to both national classification and IPC

| B. | FIELDS SEARCHED |
|---|---|

Minimum documentation searched (classification system followed by classification symbols)

G21K5/04; G21K1/093; A61N5/10; H01J37/147; H05H7/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2021-19747 A (HITACHI, LTD.) 18 February 2021 (2021-02-18)<br>paragraphs [0042], [0071], [0076], fig. 4, 9, 10 | 1-10 |
| Y | JP 2017-4711 A (NATIONAL INST. FOR QUANTUM & RADIOLOGICAL SCIENCE & TECHNOLOGY) 05 January 2017 (2017-01-05)<br>paragraphs [0050], [0054]-[0057], fig. 2 | 1-10 |

☐ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2023/028343** |

| Patent document<br>cited in search report | | | Publication date<br>(day/month/year) | Patent family member(s) | | | Publication date<br>(day/month/year) |
| --- | --- | --- | --- | --- | --- | --- | --- |
| JP | 2021-19747 | A | 18 February 2021 | US | 2022/0181042 | A1 | |
| | | | | paragraphs [0062], [0089], [0094], fig. 4, 9, 10 | | | |
| | | | | WO | 2021/020004 | A1 | |
| | | | | EP | 4006920 | A1 | |
| | | | | CN | 114080256 | A | |
| JP | 2017-4711 | A | 05 January 2017 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021019747 A